(19) Europäisches Patentamt
European Patent Office
Office européen des brevets

(11) Veröffentlichungsnummer: **0 005 246**
**A2**

(12) # EUROPÄISCHE PATENTANMELDUNG

(21) Anmeldenummer: **79101274.3**

(22) Anmeldetag: **27.04.79**

(51) Int. Cl.²: **C 07 D 307/86,** A 01 N 9/12

(30) Priorität: **10.05.78 DE 2820360**

(43) Veröffentlichungstag der Anmeldung: **14.11.79 Patentblatt 79/23**

(84) Benannte Vertragsstaaten: **BE CH DE FR GB IT NL SE**

(71) Anmelder: **BAYER Aktiengesellschaft, Zentralbereich Patente,Marken und Lizenzen Bayerwerk, D-5090 Leverkusen 1 (DE)**

(72) Erfinder: **Hartmann, Alfons, Dr., Poststrasse 35, D-6645 Beckingen (DE)**
Erfinder: **Kühle, Engelbert, Dr., von Bodelschwingh-Strasse 42, D-5060 Bergisch-Gladbach 2 (DE)**
Erfinder: **Hammann, Ingeborg, Dr., Belfortstrasse 9, D-5000 Köln 1 (DE)**

(54) **Neues N-carboxyliertes Carbamat, Verfahren zu seiner Herstellung und seine insektizide Verwendung.**

(57) Die vorliegende Erfindung betrifft den neuen N-carboxylierten N-Methyl-carbamidsäurearylester der Formel I

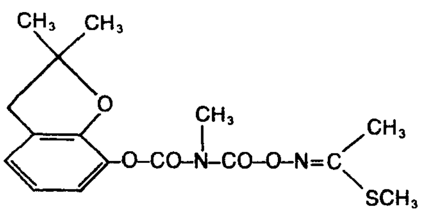

I

Er weist starke insektizide Eigenschaften auf.

Er wird erhalten, wenn man N-Chlorcarbonyl-N-methyl-2,2-dimethyl-2,3-dihydrobenzofuranyl-(7)-carbamat mit N-Hydrooxyimidothioessigsäure-S-methylester umsetzt.

EP 0 005 246 A2

0005246

BAYER AKTIENGESELLSCHAFT
Zentralbereich
Patente, Marken und Lizenzen

5090 Leverkusen, Bayerwerk
Rt/Kü
Typ Ia

Neues N-carboxyliertes Carbamat, Verfahren zu seiner Herstellung und seine insektizide Verwendung

Die vorliegende Erfindung betrifft ein neues N-carboxyliertes N-Methyl-carbamat, Verfahren zu seiner Herstellung und seine insektizide Verwendung.

Es ist bereits bekannt geworden, daß N-carboxylierte N-Methyl-carbamidsäurearylester insektizide Eigenschaften haben. (Vergl. Deutsche Offenlegungsschrift 21 32 936). Ihre Wirkung läßt jedoch, vor allem bei niedrigen Aufwandkonzentrationen, vielfach zu wünschen übrig.

Weiterhin ist das Carbamat S-Methyl-N-(methylcarbamoyloxy) thioacetamidat bekannt. Auch seine Wirkung ist nicht in allen Fällen voll befriedigend.

Es wurde nun der neue N-carboxylierte N-Methyl-carbamidsäurearylester der Formel I

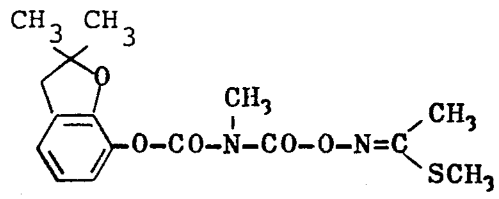 I

gefunden, der starke insektizide Eigenschaften aufweist.

Außerdem wurde gefunden, daß man die Verbindung der Formel I erhält, wenn man N-Chlorcarbonyl-N-methyl-2,2-dimethyl-2,3-dihydrobenzofuranyl-(7)-carbamat der Formel II

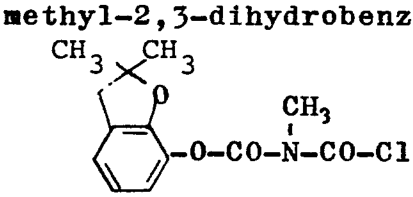 II

mit N-Hydroxyimidothioessigsäure-S-methylester der Formel III

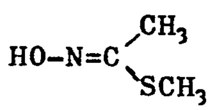 III

in Gegenwart eines säurebindenden Mittels sowie gegebenenfalls eines Verdünnungsmittels umsetzt.

Es ist ausgesprochen überraschend, daß die erfindungsgemäße Verbindung eine höhere insektizide Potenz aufweist als die eingangs erwähnten N-carboxylierten Carbamate und S-Methyl-N-(methylcarbamoyloxy)-thioacetamidat andererseits. Zu diesen genannten Wirkstoffen besteht strukturelle Verwandtschaft. Der neue Stoff stellt somit eine wertvolle Bereicherung der Technik dar.

Der Reaktionsablauf kann durch das folgende Formelschema wiedergegeben werden:

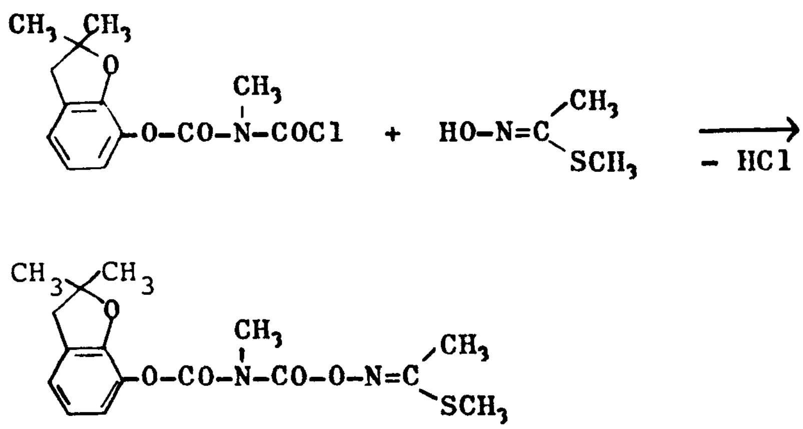


Die Ausgangskomponente II kann nach dem in der DE-OS 2 142 496 beschriebenen Verfahren hergestellt und entweder in reiner Form (Fp. $63^{o}$) gewonnen oder im anfallenden Reaktionsgemisch ohne Isolierung weiter umgesetzt werden.

Die Ausgangskomponente III ist in der DE-OS 1 568 646 beschrieben.

Als Verdünnungsmittel für die erfindungsgemäße Darstellung eignen sich alle inerten organischen Lösungsmittel. Hierzu gehören Ether wie Diethylether, Dioxan oder Tetrahydrofuran, Kohlenwasserstoffe wie Benzol oder Toluol, Chlorkohlenwasserstoffe wie Dichlormethan, Chloroform oder Chlorbenzol, weiterhin Nitrile, Ester, Ketone sowie Gemische aus diesen Lösungsmitteln.

Als säurebindendes Mittel setzt man dem Reaktionsgemisch vorzugsweise eine tertiäre organische Base wie z. B. Triethylamin oder Benzyldimethylamin zu.

Die Reaktionstemperatur kann in einem größeren Bereich variiert werden. Im allgemeinen arbeitet man zwischen

0 und $100^{o}$, vorzugsweise bei 10 bis $60^{o}$.

Gewöhnlich werden die Reaktionspartner in äquimolaren Mengen eingesetzt.

Der Wirkstoff eignet sich bei guter Pflanzenverträglichkeit und günstiger Warmblütertoxizität zur Bekämpfung von tierischen Schädlingen, insbesondere Insekten, die in der Landwirtschaft, in Forsten, im Vorrats- und Materialschutz sowie auf dem Hygienesektor vorkommen. Er ist gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophaus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Der Wirkstoff kann in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung des erfindungsgemäßen Wirkstoffes erfolgt in Form seiner handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung des erfindungsgemäßen Wirkstoffes geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

Beispiel A

Plutella-Test (Wirkungsdauer nach Spritzen)

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlpflanzen (Brassica oleracea) von ca. 10 - 15 cm Höhe werden mit der gewünschten Wirkstoffzubereitung tropfnaß gespritzt.

Nach der gewünschten Zeit werden die Pflanzen mit Raupen der Kohlschabe (Plutella maculipennis) besetzt. Nach jeweils 3 Tagen wird die Abtötung in % bestimmt. Dabei bedeuten 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1

Beispiel B

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1

Herstellungsbeispiel

10,1 g (0,1 Mol) Triethylamin werden unter Rühren zu 28,35 g (0,1 Mol) N-Chlorcarbonyl-N-methyl-2,2-dimethyl-2,3-dihydrobenzofuranyl-(7)-carbamat und 10,5 g (0,1 Mol) N-Hydroxyimidothioessigsäure-S-methylester in 400 ml Toluol getropft. Man rührt 5 Stdn. bei Raumtemperatur und 1/2 Stde. bei 50°, schüttelt mit Wasser aus, trocknet die org. Phase über Natriumsulfat und dampft sie am Rotationsverdampfer ein. Das zurückbleibende Oel kristallisiert nach einigen Stunden. Man verrührt mit wenig Ether/Petrolether (1 : 1) und erhält nach Absaugen 36,4 g farblose, DC-chromatographisch reine Kristalle vom Fp. 90°.

Die Ausgangsverbindung kann wie folgt hergestellt werden:

Zu einer Lösung von 65,6 g (0,4 Mol) 2,2-Dimethyl-2,3-dihydrobenzofuranol-(7) und 62,4 g (0,4 Mol) N-Bis-chlorcarbonyl-methylamin in 1,2 ltr. Toluol tropft man unter Rühren 40,4 g (0,4 Mol) Triethylamin. Man rührt 8 Stdn. bei Raumtemperatur, saugt das ausgefallene Amin-hydrochlorid ab und dampft i. Vak. ein. Der Rückstand kristallisiert nach kurzer Zeit. Er wird mit Petrolether verrührt und abgesaugt. 113,4 g farblose Kristalle, Fp. 63°.

Patentansprüche

1. Verbindungen der Formel I

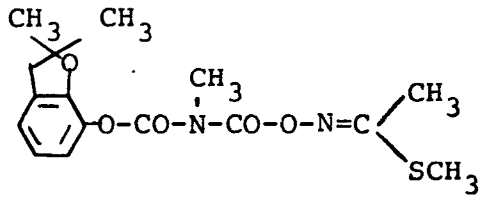

I

2. Verfahren zur Herstellung der Verbindung der Formel I gemäß Anspruch 1, dadurch gekennzeichnet, daß man N-Chlorcarbonyl-N-methyl-2,2-dimethyl-2,3-dihydrobenzofuranyl-(7)-carbamat der Formel II

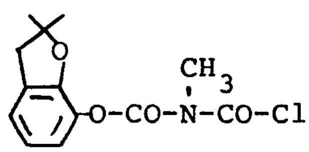

II

mit N-Hydroxyimidothioessigsäure-S-methylester der Formel III

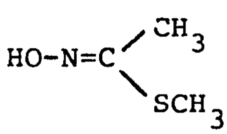

III

in Gegenwart eines säurebindenden Mittels sowie gegebenenfalls eines Verdünnungsmittels umsetzt.

3. Insektizide Mittel, gekennzeichnet durch einen Gehalt an der Verbindung der Formel (I) gemäß Anspruch 1.

4. Verwendung der Verbindung der Formel (I) gemäß Anspruch 1 zur Bekämpfung von Insekten.

5. Verfahren zur Bekämpfung von Insekten, dadurch gekennzeichnet, daß man die Verbindung der Formel (I) gemäß Anspruch 1 auf Insekten und/oder ihren Lebensraum einwirken läßt.

6. Verfahren zur Herstellung insektizider Mittel, dadurch gekennzeichnet, daß man die Verbindung der Formel (I) gemäß Anspruch 1 mit Streckmitteln und/oder oberflächenaktiven Mitteln vermischt.